# EUROPEAN PATENT SPECIFICATION

(11) **EP 1 897 558 B1**
(45) Date of publication and mention of the grant of the patent: **04.09.2013**
(21) Application number: 06747305.8
(22) Date of filing: 09.06.2006
(51) Int. Cl.: A61K 47/38, A61K 9/14, A61K 9/20, A61K 9/36, A61K 31/536, A61K 45/00, A61K 47/10, A61K 47/26, A61P 1/00, A61P 3/04, A61P 3/06, A61P 3/10, A61P 9/00, A61P 9/10, A61P 9/12, A61P 43/00

(54) **SOLID PREPARATION OF 2-HEXADECYLOXY-6-METHYL-4H-3,1-BENZOXAZIN-4-ONE**
FESTE ZUBEREITUNG MIT 2-HEXADECYLOXY-6-METHYL-4H-3,1-BENZOXAZIN-4-ON
PREPARATION SOLIDE DE 2-HEXADECYLOXY-6-METHYL-4H-3,1-BENZOXAZIN-4-ONE

(30) Priority: 09.06.2005 JP 2005170172
(43) Date of publication of application: 12.03.2008
(73) Proprietor: Norgine BV, 1101 CA Amsterdam Zuid-Oost (NL)
(72) Inventor: SUZUKI, H., c/o TAKEDA PHARMACEUTICAL COMPANY LTD, Osaka-shi, Osaka 532-8686 (JP); EBISAWA, Y., c/o TAKEDA PHARMACEUTICAL COMPANY LTD, Osaka-shi, Osaka 532-8686 (JP); YOSHINARI, T., TAKEDA PHARMACEUTICAL COMPANY LTD, Osaka-shi, Osaka 532-8686 (JP)
(74) Representative: Crooks, Elizabeth Caroline
(86) International application number: PCT/JP2006/312078
(87) International publication number: WO 2006/132440

(56) References cited:
- EP-A1- 1 203 580
- EP-A2- 0 882 450
- WO-A1-00/09122
- WO-A1-00/78292
- WO-A1-01/19340
- WO-A1-98/34607
- WO-A1-02/098412
- WO-A1-2004/060401
- WO-A2-02/09815
- WO-A2-2004/024128
- JP-A- 07 165 568
- JP-A- 10 182 436
- JP-A- 2000 273 039
- JP-A- 2000 344 660
- JP-A- 2001 316 249
- JP-A- 2001 335 469
- US-A- 5 643 874
- US-A1- 2003 158 232
- US-A1- 2005 003 001

## Description

### Technical Field

The present invention relates to a solid preparation. More particularly, the present invention relates to a solid preparation superior in the stability during production and during preservation of a poorly water-soluble substance having a low melting point, as well as disintegration property of the preparation and release property of a poorly water-soluble substance having a low melting point, after oral administration of the preparation.

### Background Art

Solid formulations such as a pharmaceutical product containing, as an active ingredient, a poorly water-soluble substance having a low melting point and the like show unsuitable properties during production and during preservation, such as coagulation, melting, melt adhesion and the like. Thus, various formulation methods to overcome such unsuitable properties have been considered heretofore.

For example, a production method of a solid preparation, comprising heat-melting cyclandelate, which is a poorly water-soluble substance having a low melting point, and adding ultrafine synthetic aluminum silicate and/or silicon dioxide in an amount free of providing drug efficacy has been reported (see JP-B-51-16491).

### Disclosure of the Invention

However, a solid preparation containing a large amount of a poorly water-soluble substance having a low melting point is difficult to obtain because adsorbent and excipient are used in large amounts according to the above-mentioned known technique.

The present inventors have studied various preparation compositions in an attempt to solve this problem and found a solid preparation free of coagulation, melting, melt adhesion and the like during production and preservation even when a poorly water-soluble substance having a low melting point is contained in a large amount, which formulation is superior in stability and, after oral administration, retracts water rapidly, swells and is disintegrated in the gastrointestinal tract to quickly release a poorly water-soluble substance having a low melting point. Based thereon, the solid preparation of the present invention has been completed.

Accordingly, the present invention relates to the following [1] to [17].
[1] A solid preparation having the following characteristics 1) to 3):
   1) containing a poorly water-soluble substance having a low melting point, a saccharide, and a cellulose selected from a crystalline cellulose and a low-substituted hydroxypropylcellulose,
   2) a saccharide/cellulose weight ratio exceeding 2,
   3) a cellulose content of not less than 5 wt%.
[2] The solid preparation of the above-mentioned [1], wherein the poorly water-soluble substance having a low melting point has water solubility at 37°C of not more than 10 mg/L.
[3] The solid preparation of the above-mentioned [1], wherein the poorly water-soluble substance having a low melting point has a melting point of 10 to 100°C.
[4] The solid preparation of the above-mentioned [1], wherein the poorly water-soluble substance having a low melting point has an average particle size of 1 to 100 µm.
[5] The solid preparation of the above-mentioned [1], wherein the poorly water-soluble substance having a low melting point is a lipase inhibitor.
[6] The solid preparation of the above-mentioned [5], wherein the lipase inhibitor is 2-hexadecyloxy-6-methyl-4H-3,1-benzoxazin-4-one or a salt thereof.
[7] The solid preparation of the above-mentioned [1], wherein the saccharide is a sugar alcohol.
[8] The solid preparation of the above-mentioned [7], wherein the sugar alcohol is mannitol.
[9] The solid preparation of the above-mentioned [1], wherein the cellulose is a crystalline cellulose.
[10] The solid preparation of the above-mentioned [1], wherein the content of the poorly water-soluble substance having a low melting point is 5 to 60 wt%.
[11] The solid preparation of the above-mentioned [1], wherein the content of the saccharide is 30 to 75 wt%.
[12] The solid preparation of the above-mentioned [1], wherein the content of the cellulose is 5 to 15 wt%.
[13] The solid preparation of the above-mentioned [1], wherein the weight ratio of saccharide/cellulose is 3 to 9.
[14] The solid preparation of the above-mentioned [1], which is a tablet.
[15] The solid preparation of the above-mentioned [1], which has a disintegration time in water at 37°C of within 30 min.
[16] A production method of the solid preparation of the above-mentioned [1], which comprises granulating a mixture of a poorly water-soluble substance having a low melting point, a saccharide and a cellulose selected from a crystalline cellulose and a low-substituted hydroxypropylcellulose.
[17] The method of the above-mentioned [16], wherein the granulation is performed using a fluidized bed granulator.

### Effect of the Invention

In the solid preparation of the present invention, coagulation, melting, melt adhesion and the like of a poorly water-soluble substance having a low melting point, which are generally observed during production and preservation, are suppressed. Therefore, the solid preparation of the present invention is superior in the disintegration property and release property of the poorly water-soluble substance having a low melting point, after oral administration.

Moreover, the solid preparation of the present invention is superior in the stability during production and preservation even when a poorly water-soluble substance having a low melting point is contained in a large amount, and also superior in the disintegration property and release property of the poorly water-soluble substance having a low melting point, after oral administration.

Since the production method of the present invention can be performed under temperature conditions at not more than the melting point of the poorly water-soluble substance having a low melting point, the poorly water-soluble substance having a low melting point does not require heat-melting. Therefore, the production method of the present invention does not disintegrate a poorly water-soluble substance having a low melting point and is extremely useful as a convenient production method of a solid preparation.

### Best Mode for Embodying the Invention

The present invention is explained in detail in the following.

The solid preparation of the present invention is characterized by the following 1) to 3).
1) containing a poorly water-soluble substance having a low melting point, a saccharide, and a cellulose selected from a crystalline cellulose and a low-substituted hydroxypropylcellulose,
2) a saccharide/cellulose weight ratio exceeding 2,
3) a cellulose content of not less than 5 wt%.

The poor water solubility of the poorly water-soluble substance having a low melting point to be used for the solid preparation of the present invention means the property associated with difficulty in dissolution in water. In the present invention, for example, the solubility of a poorly water-soluble substance having a low melting point in water at 37°C is generally not more than 10 mg/L, preferably not more than 1 mg/L, more preferably not more than 0.5 mg/L.

As used herein, the solubility is determined as follows. First, an excess amount of a poorly water-soluble substance having a low melting point substance is added to purified water (5 ml). The obtained mixture is stood still in a thermostatic tank at 37°C for 30 min, and stirred in a voltex mixer. The operation of standing still and stirring is repeated 3 more times, and the obtained suspension is filtered through a syringe filter (manufactured by Japan Pall, trade name: Acrodisc LC25, PVDF, pore size 0.2 µm). The concentration (mg/L) of the poorly water-soluble substance having a low melting point in the thus-obtained filtrate is taken as the solubility.

In addition, the low melting point of a poorly water-soluble substance having a low melting point means a melting point within the range of generally 10°C to 100°C, preferably 20°C to 90°C, more preferably 30°C to 90°C. The melting point can be measured, for example, according to the melting point measurement method defined in the Japanese Pharmacopoeia.

The average particle size of the above-mentioned poorly water-soluble substance having a low melting point is generally 1 µm to 100 µm, preferably 1 µm to 70 µm, more preferably 1 µm to 60 µm, particularly preferably 10 µm to 50 µm. In the present specification, the average particle size means a cumulative 50% particle size (X50) measured using a dry laser diffractometer (HELOS, Sympatec GmbH).

The content of the above-mentioned poorly water-soluble substance having a low melting point in a solid preparation is generally 5 wt% to 60 wt%, preferably 5 wt% to 55 wt%, more preferably 5 wt% to 50 wt%.

Examples of the above-mentioned poorly water-soluble substance having a low melting point include lipase inhibitors, anti-inflammatory agents (e.g., ibuprofen, ketoprofen), electron transport chain coenzyme agents (e.g., coenzyme Q10, idebenone) and the like, with preference given to lipase inhibitors.

Examples of lipase inhibitors include orlistat, and the following compound described in US Patent No. 6624161: 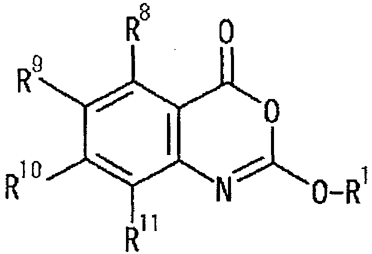 wherein R¹ is branched or non-branched C₁₀₋₂₀ alkyl (said C₁₀₋₂₀ alkyl may be interrupted with 1 or 2 oxygen atoms, and optionally substituted by one or more substituents selected from aryl, aryloxy, heteroaryl, heteroaryloxy, cyano, nitro, -CO₂R³, - NR³R⁴, -CONR³R⁴, OH and halogen atom, R³ and R⁴ are each independently hydrogen atom or C₁₋₆ alkyl, R⁸, R⁹, R¹⁰ and R¹¹ are each independently hydrogen atom, halogen atom, hydroxy, amino, nitro, cyano, thiol, C₁₋₁₀ alkyl, C₁₋₁₀ alkoxy, C₃₋₁₀ cycloalkyl, C₃₁₀ cycloalkoxy, C(O)R⁵, C(O)NR⁵R⁶, S(O)R⁵ or haloC₁₋₁₀ alkyl, and R⁵ and R⁶ are each independently hydrogen atom or C₁₋₁₀ alkyl.

Of the above-mentioned compounds, 2-hexadecyloxy-6-methyl-4H-3,1-benzoxazin-4-one (hereinafter to be simply referred to as compound A) is preferable.

The poorly water-soluble substance having a low melting point to be used for the solid preparation of the present invention may be a salt. Examples of such salt include metal salt, ammonium salt, a salt with organic base, a salt with inorganic acid, a salt with organic acid, a salt with basic or acidic amino acid and the like. Preferable examples of the metal salt include alkali metal salt such as sodium salt, potassium salt and the like; alkaline earth metal salt such as calcium salt, magnesium salt, barium salt and the like; aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic base include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysin, ornithine and the like, and preferable examples of the salt with acidic amino acid include salts with aspartic acid, glutamic acid and the like.

Of these, pharmaceutically acceptable salts are preferable.

Examples of the saccharides to be used for the solid preparation of the present invention include sugar alcohols (e.g., mannitol (mannit), erythritol, xylitol, maltitol, sorbitol), disaccharides (e.g., maltose, sucrose, cellobiose, lactose), monosaccharides (e.g., arabinose, xylose, ribose, 2-deoxyribose, glucose, fructose, galactose, mannose, sorbose, rhamnose, fucose), oligosaccharides (e.g., malttriose, raffinose sugar, stachyose) and the like. From the aspect of disintegration property of a solid preparation and release property of a poorly water-soluble substance having a low melting point from the solid preparation, preferred are sugar alcohols and more preferred is mannitol.

The content range of the saccharides in the solid preparation of the present invention is generally 10 wt% to 75 wt%, preferably 20 wt% to 75 wt%, more preferably 30 wt% to 75 wt%. When saccharides are contained within such ranges, the rate of penetration of water into a solid preparation increases, and disintegration property of a solid preparation and release property of a poorly water-soluble substance having a low melting point are improved.

The cellulose to be used for the solid preparation of the present invention is selected from a crystalline cellulose and a low-substituted hydroxypropylcellulose. From the aspect of rapid release of a poorly water-soluble substance having a low melting point from a solid preparation, a crystalline cellulose is preferable.

Also from the aspect of improved forming property of a solid preparation, a crystalline cellulose is preferably as the cellulose.

Here, the improved forming property of a solid preparation means that the solid preparation can maintain practically sufficient hardness even when the pressure necessary during forming is attenuated to decrease the compression density of the solid preparation.

In the solid preparation of the present invention, coagulation, melting, melt adhesion and the like of a poorly water-soluble substance having a low melting point, which are developed during the production process of the solid preparation, can be prevented by the use of crystalline cellulose. Such preventive effect can be remarkably afforded for a solid preparation having a high content of a poorly water-soluble substance having a low melting point (e.g., solid preparation wherein the content of a poorly water-soluble substance having a low melting point in the solid preparation is not less than 20 wt%).

Particularly, during production of a solid preparation having a high content of a poorly water-soluble substance having a low melting point (e.g., solid preparation wherein the content of a poorly water-soluble substance having a low melting point in the solid preparation is not less than 20 wt%), coagulation, melting, melt adhesion and the like of the poorly water-soluble substance having a low melting point are highly likely developed. Therefore, the solid preparation of the present invention capable of affording sufficient hardness of the solid preparation even when the pressure necessary during forming is attenuated to decrease the compression density and increase the void percentage of the solid preparation can provide a remarkable preventive effect on the coagulation, melting, melt adhesion and the like of a poorly water-soluble substance having a low melting point.

In the solid preparation of the present invention, moreover, the coagulation, melting, melt adhesion and the like of a poorly water-soluble substance having a low melting point, which are developed during the preservation process of the solid preparation, can be prevented by the use of crystalline cellulose.

Particularly, during preservation of a solid preparation having a high content of a poorly water-soluble substance having a low melting point (e.g., solid preparation wherein the content of a poorly water-soluble substance having a low melting point in the solid preparation is not less than 20 wt%), coagulation, melting, melt adhesion and the like of the poorly water-soluble substance having a low melting point are highly likely developed. Therefore, the solid preparation of the present invention can provide a remarkable preventive effect on the coagulation, melting, melt adhesion and the like of a poorly water-soluble substance having a low melting point.

In the preparation of the present invention, the weight ratio of the above-mentioned saccharide/cellulose exceeds 2, preferably within the range of more than 2 up to 15, more preferably within the range of 3 to 9, most preferably 4 to 7. A weight ratio of the saccharide/cellulose of not less than 15 is not preferable because the rate of water uptake by the solid preparation becomes insufficient. On the other hand, when the weight ratio of the above-mentioned saccharide/cellulose is not more than 2, the solid preparation generally absorbs moisture to result in difficult handling. Generally, tablet thickness is controlled in a tabletting step. When the tablet swells after tabletting to increase the tablet thickness, a problem occurs in that a control to the tablet thickness for quality management becomes difficult. Particularly, when a film coating step is involved and the tablet (plain tablet) become swollen, concaves and convexes are formed on the surface of the tablet, thus giving rise to a quality problem. Moreover, since the solid preparation per se becomes fragile, the quality problems of crack, chip and the like possibly occur. Particularly, when a film coating step is involved, a quality problem of crack, chip or the like even in a slight number of tablets adversely affects the whole production batch.

In the solid preparation of the present invention, the content of the cellulose is generally not less than 5 wt%, preferably 5 wt% to 30 wt%, more preferably 5 wt% to 25 wt%, particularly preferably 5 wt% to 15 wt%. In the solid preparation of the present invention, when the content of the cellulose is less than 5 wt%, unpreferably, the object disintegration property and release property cannot be achieved. On the other hand, when the content of the cellulose in the solid preparation of the present invention exceeds 30 wt%, the obtained solid preparation swells and becomes difficult to handle. In addition, since the solid preparation per se becomes fragile, the quality problems of crack, chip and the like possibly occur.

In the solid preparation of the present invention, the weight ratio of the poorly water-soluble substance having a low melting point and saccharide is 1:50 to 50:1, preferably 1:15 to 10:1, more preferably 1:15 to 5:1.

The dosage form of the solid preparation of the present invention is, for example, granule, pill, tablet, capsule and the like, with preference given to tablet. The shape of the tablet is not particularly limited, and may be a plain tablet of round tablet, oval tablet, oblong tablet and the like, a coated tablet thereof and the like. Moreover, the solid preparation of the present invention may be a tablet comprising separated groups, which is obtained by mixing two or more kinds of granules and tabletting the mixture; a multi-layer tablet such as a two-layer tablet, a three-layer tablet and the like; a nucleated tablet; a press-coated tablet and the like.

In addition, the solid preparation of the present invention may contain, in addition to the above-mentioned poorly water-soluble substance having a low melting point, a saccharide and a cellulose selected from a crystalline cellulose and a low-substituted hydroxypropylcellulose, various additives such as a pharmaceutically acceptable carrier, specifically, excipient, disintegrant, binder, lubricant, colorant, flavor, light shielding agent, plasticizer, stabilizer and the like generally used for pharmaceutical preparations, within the range that does not impair the disintegration property of the solid preparation and release property of the poorly water-soluble substance having a low melting point from the solid preparation.

Examples of the excipient include light anhydrous silicic acid, magnesium carbonate, calcium carbonate, calcium phosphate, calcium sulfate, aluminum silicate, aluminum metasilicate and the like.

Examples of the disintegrant include carmellose calcium, croscarmellose sodium, carboxymethyl starch sodium, crosslinked insoluble polyvinylpyrrolidone and the like.

Examples of the binder include hydroxypropylcellulose, hydroxypropylmethylcellulose, pregelatinized starch, gelatin, gum arabic powder, polyvinylpyrrolidone, dextrin, pullulan and the like.

Examples of the lubricant include stearic acid, calcium stearate, magnesium stearate, talc, colloidal silica and the like.

Examples of the colorant include yellow ferric oxide, diiron trioxide and the like.

The flavor may be a synthetic substance or a naturally occurring substance and examples thereof include lemon flavor, lime flavor, orange flavor, strawberry flavor, menthol and the like.

Examples of the light shielding agent include titanium oxide, talc, calcium carbonate, magnesium carbonate and the like.

Examples of the plasticizer include polyethylene glycol (macrogol), propylene glycol, copolyvidone and the like.

Examples of the stabilizer include ascorbic acid, ascorbic acid sodium, erysorbic acid and the like.

When a lipase inhibitor is used as a poorly water-soluble substance having a low melting point, the solid preparation of the present invention may contain, where necessary, the oil adsorbent (e.g., methylcellulose, xanthane gum) described in WO 00/09122 and the like.

### [Production method]

The solid preparation of the present invention can be produced by combining known methods employed for each dosage form. The conditions of each step can be determined according to a conventional method.

Preferably, the solid preparation of the present invention is produced by a method comprising granulating a mixture of a poorly water-soluble substance having a low melting point, a saccharide and a cellulose selected from a crystalline cellulose and a low-substituted hydroxypropylcellulose (hereinafter sometimes to be abbreviated as the production method of the present invention). The granulation can be performed using any granulator conventionally employed (e.g., fluidized bed granulator, high speed mixer, kneader). However, for prevention of denaturation of a poorly water-soluble substance having a low melting point, a fluidized bed granulator is preferably used for granulation.

The above-mentioned granulation is preferably performed under temperature conditions under which the product temperature in the granulation step is not more than the melting point of the poorly water-soluble substance having a low melting point to be used. When a poorly water-soluble substance having a low melting point and an excipient are co-present, the melting point of the poorly water-soluble substance having a low melting point may become lower than the general melting point. In this case, the granulation is preferably performed at a product temperature controlled to not higher than the decreased melting point. The amounts of the poorly water-soluble substance having a low melting point, saccharide and cellulose are as mentioned above.

The granules to be obtained by the above-mentioned granulation contain 50 µm to 1.5 mm particles in a proportion of not less than 50% (preferably 150 µm to 1.0 mm particles in a proportion of not less than 50%). The obtained granules may be dried as necessary for about 0.01 hr to 72 hr to remove water. In addition, the obtained granules may be further sized as necessary. For sizing, a commercially available granulator such as a power mill and the like is generally used. The granules after sizing contain about 50 µm to 1.5 mm particles in a proportion of not less than 50% (preferably 150 µm to 1.0 mm particles in a proportion of not less than 50%). Moreover, a disintegrant such as croscarmellose sodium and the like and a lubricant such as magnesium stearate and the like may be added thereto. For mixing them, a commercially available mixer such as a tumbler mixer and the like is generally used. The content of the disintegrant and lubricant to be used is about 0.1 wt% to 25 wt% and about 0.1 wt% to 10 wt%, respectively.

While the obtained granules may be directly used as a granule agent, they are generally formed in a dosage form of pill, tablet, capsule and the like.

For formation of a tablet, for example, a commercially available forming machine such as tableting machine and the like is used. The tabletting pressure for forming a tablet is generally about 1 kN to 25 kN. The round tablet generally has a diameter of about 5 mm to 20 mm, and a thickness of about 1 mm to 10 mm. The oval tablet generally has a long diameter of about 7 mm to 20 mm, a short diameter of about 5 mm to 15 mm, and a thickness of about 1 mm to 10 mm. The oblong tablet generally has a long diameter of about 7 mm to 20 mm, a short diameter of about 5 mm to 15 mm, and a thickness of about 1 mm to 10 mm.

The tablet obtained above may be subjected to coating such as film coating and the like to give various coated preparations such as a film-coated tablet and the like.

For the film coating operation, a pan coating apparatus and the like are generally used. Examples of the film-coated tablet include a film-coated round tablet, a film-coated oval tablet, and a film-coated oblong tablet.

The film coating liquid to be used for the above-mentioned film coating can be prepared, for example, by dissolving or suspending a film coating polymer such as hydroxypropylmethylcellulose and the like in, for example, a solvent such as water and the like. The film coating liquid preferable further contains a colorant, a light shielding agent and the like. The product (tablet) temperature during spraying a film coating liquid is generally controlled to about 10°C to 100°C, more preferably to about 30°C to 80°C, and still more preferably to about 35°C to 60°C.

Since the production method of the present invention can be performed under temperature conditions not higher than the melting point of a poorly water-soluble substance having a low melting point, the poorly water-soluble substance having a low melting point does not need to be melted by heating. Therefore, the production method of the present invention is extremely useful as a convenient production method to afford a solid preparation without decomposition of the poorly water-soluble substance having a low melting point.

The solid preparation of the present invention obtained as mentioned above has the desired disintegration property. Specifically, for example, the disintegration time in water at 37°C is generally within 30 min, preferably within 20 min, more preferably within 10 min.

The solid preparation of the present invention obtained as mentioned above is superior in the disintegration property and release property of a poorly water-soluble substance having a low melting point after oral administration.

Particularly, since a lipase inhibitor such as compound A has an antiobesity action but shows low toxicity and is safe, when the solid preparation of the present invention contains a lipase inhibitor as a poorly water-soluble substance having a low melting point, the solid preparation can be used as a safe agent for the prophylaxis or treatment of various diseases such as obesity, hyperlipidemia (e.g., hypertriglyceridemia, hypercholesterolemia, hypoHDL-emia, postprandial hyperlipemia), hyperglycemia (type 2 diabetes, impaired glucose tolerance), hypertension, cardiovascular disease, apoplexy, gastrointestinal diseases and the like or complications of these diseases (e.g., obesity occurring in association with type 2 diabetes, obesity occurring in association with hyperlipidemia, metabolic syndrome) in mammals (e.g., human, rat, mouse, cat, dog, rabbit, cattle, swine, hamster, sheep, monkey).

While the dose of the solid preparation of the present invention varies depending on the kind and content of a poorly water-soluble substance having a low melting point, dosage form, duration of drug release, administration subject animal (e.g., mammals such as human, rat, mouse, cat, dog, rabbit, cattle, swine, hamster, sheep, monkey and the like), administration object, symptom, age of patients and the like, for example, it is generally about 1 mg to 500 mg per day for oral administration to an adult patient (body weight: 60 kg).

Moreover, for example, for oral administration of a solid preparation containing a lipase inhibitor (preferably compound A) as a poorly water-soluble substance having a low melting point to an adult patient (body weight: 60 kg) affected with obesity or complications thereof (e.g., obesity occurring in association with type 2 diabetes, obesity occurring in association with hyperlipidemia), the solid preparation of the present invention containing about 1 mg to 500 mg, preferably about 5 mg to 250 mg, still more preferably about 5 mg to 100 mg, of a poorly water-soluble substance having a low melting point can be administered in 1 to 3 portions for one day.

Where necessary, the solid preparation of the present invention may contain a drug other than the poorly water-soluble substance having a low melting point, within the range that does not impair the release property, or may be used in combination with other drugs.

Examples of the drug (hereinafter to be abbreviated as a concomitant drug) that can be added to the solid preparation of the present invention along with a poorly water-soluble substance having a low melting point or used in combination with the solid preparation of the present invention include the following.

### (1) Therapeutic agents for diabetes mellitus

Insulin preparations [e.g., animal insulin preparations extracted from the bovine or swine pancreas; human insulin preparations synthesized by a genetic engineering technique using Escherichia coli or a yeast; zinc insulin; protamine zinc insulin; fragment of insulin or derivatives thereof (e.g., INS-1); oral insulin preparations], agents for improving insulin resistance (e.g., pioglitazone or salts thereof (preferably hydrochloride), rosiglitazone or salts thereof (preferably maleate), Reglixane, Netoglitazone, FK-614, Rivoglitazone, DRF-2593, Edaglitazone (BM-13.1258), R-119702, compounds described in WO01/38325, Tesaglitazar, Ragaglitazar, Muraglitazar, ONO-5816, LM-4156, Metaglidasen (MBX-102), Naveglitazar (LY-519818), MX-6054, LY-510929, (Balaglitazone), T-131 or salts thereof, THR-0921), α-glucosidase inhibitors (e.g., voglibose, acarbose, miglitol, emiglitate), biguanides (e.g., phenformin, metformin, buformin or salts thereof (e.g., hydrochloride, fumarate, succinate)), insulin secretagogues (sulfonylureas (e.g., tolbutamide, glibenclamide, gliclazide, chlorpropamide, tolazamide, acetohexamide, glyclopyramide, glimepiride, glipizide, glybuzole), repaglinide, senaglinide, nateglinide, mitiglinide or its calcium salt hydrate), GPR40 agonist, GLP-1 receptor agonist [e.g., GLP-1, GLP-1MR agent, NN-2211, AC-2993 (exendin-4), BIM-51077, Aib(8,35)hGLP-1(7,37)NH₂, CJC-1131], dipeptidylpeptidase IV inhibitors (e.g., NVP-DPP-278, PT-100, P32/98, Vildagliptin (LAF-237), P93/01, TS-021, (Sitagliptin phosphate) (MK-431), Saxagliptin (BMS-477118), E-3024, T-6666 (TA-6666), 823093, 825964, 815541), β3 agonists (e.g., AJ-9677), amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLT (sodium-glucose cotransporter) inhibitors (e.g., T-1095), 11β-HSD1 inhibitors (e.g., BVT-3498), adiponectin or agonists thereof, IKK inhibitors (e.g., AS-2868), leptin resistance improving agent, somatostatin receptor agonist (e.g., compounds described in WO01/25228, WO03/42204, WO98/44921, WO98/45285, WO99/22735), glucokinase-activating agent (e.g., Ro-28-1675), and the like.

### (2) Therapeutic agents for diabetic complications

Aldose reductase inhibitors (e.g., tolrestat, epalrestat, zenarestat, zopolrestat, fidarestat, minalrestat, ranirestat, CT-112), neurotrophic factors and agents for increasing them (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), agents for accelerating nerve regeneration (e.g., Y-128, VX853, prosaptide), PKC inhibitors (e.g., ruboxistaurin mesylate), AGE inhibitors (e.g., ALT-945, pimagedine, N-phenacylthiazolium bromide (ALT-766), EXO-226, ALT-711, Pyridorin, pyridoxamine), reactive oxygen scavengers (e.g., thioctic acid), cerebral vasodilators (e.g., tiapuride), somatostatin receptor agonists (e.g., BIM23190), apoptosis signal regulating kinase-1 (ASK-1) inhibitors, and the like.

### (3) Antihyperlipidemic agents

HMG-CoA reductase inhibitors (e.g., pravastatin, simvastatin, lovastatin, atorvastatin, fluvastatin, pitavastatin, rosvastatin or salts thereof (e.g., sodium salt, potassium salt)), squalene synthetase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., cholestyramine), probucol, nicotinic acid drugs (e.g., nicomol, niceritrol), ethyl eicosapentaenoate, vegetable sterol (e.g., soysterol, γ-oryzanol), and the like.

### (4) Hypotensive agents

Angiotensin converting enzyme inhibitors (e.g., captopril, enalapril, delapril, lisinopril), angiotensin II antagonists (e.g., losartan, candesartan cilexetil, eprosartan, valsartan, termisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121, etc.), α1 blocker (e.g., prazosin chloride, terazosin chloride, bunazosin chloride), β blocker (e.g., propranolol chloride, pindolol, atenolol, celiprolol chloride, metoprolol tartrate), α1 and β blocker (e.g., labetalol hydrochloride, carvedilol, bunitrolol hydrochloride), clonidine, and the like.

### (5) Antiobesity agents

Antiobesity drugs acting on the central nervous system (e.g., dexfenfluramine, fenfluramine, phentermine, sibutramine, anfepramone, dexamphetamine, mazindol, phenylpropanolamine, clobenzorex; MCH receptor antagonists (e.g., compounds disclosed in SB-568849; SNAP-7941; WO01/82925 and WO01/87834); neuropeptide Y antagonists (e.g., CP-422935); cannabinoid receptor antagonists (e.g., SR-141716, SR-147778); ghrelin antagonists), β3 agonists (e.g., AJ-9677), anorectic peptides (e.g., leptin, CNTF (Ciliary Neurotrophic Factor)), cholecystokinin agonists (e.g., lintitript, FPL-15849), feeding deterrent (e.g., P-57), and the like.

### (6) Diuretic agents

Xanthine derivatives (e.g., sodium salicylate theobromine, calcium salicylate theobromine), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide), antialdosterone preparations (e.g., spironolactone, triamterene), carbonic anhydrase inhibitors (e.g., acetazolamide), chlorobenzenesulfonamide preparations (e.g., chlorthalidone, mefruside, indapamide), azosemide, isosorbide, ethacrynic acid, piretanide, bumetanide, furosemide, meticrane, and the like.

### (7) Chemotherapeutic agents

Alkylating agents (e.g., cyclophosphamide, ifosphamide), metabolic antagonists (e.g., methotrexate, 5-fluorouracil or derivatives thereof (e.g., furtulon, neofurtulon)), antitumor antibiotics (e.g., mitomycin, adriamycin), plant-derived antitumor agents (e.g., vincristine, vindesine, taxol), cisplatin, carboplatin, etoposide and the like.

### (8) Immunotherapeutic agents

Microorganism or bacterium components (e.g., muramyl dipeptide derivatives, Picibanil), immunopotentiator polysaccharides (e.g., lentinan, schizophyllan, krestin), genetically engineered cytokines (e.g., interferons, interleukins (e.g., IL-1, IL-2, IL-12)), colony stimulating factors (e.g., granulocyte colony stimulating factor, erythropoietin) and the like.

### (9) Antithrombotic agents

Heparin (e.g., heparin sodium, heparin calcium, dalteparin sodium), warfarin (e.g., warfarin potassium), antithrombins (e.g., argatroban), thrombolytic agent (e.g., urokinase, tisokinase, alteplase, nateplase, monteplase, pamiteplase), platelet aggregation inhibitors (e.g., ticlopidine hydrochloride, cilostazol, ethyl eicosapentaenoate, beraprost sodium, sarpogrelate hydrochloride), and the like.

### (10) Cachexia improving agents

Progesterone derivatives (e.g., megestrol acetate), metoclopramide drugs, tetrahydrocannabinol drugs, fat metabolism ameliorating agents (e.g., eicosapentaenoic acid), growth hormones, IGF-1, and antibodies to the cachexia-inducing factors TNF-α, LIF, IL-6, oncostatin M, and the like.

### (11) Anti-inflammatory agents

Steroids (e.g., dexamethazone), sodium hyaluronate, cyclooxygenase inhibitor (e.g., indomethacin, ketoprofen, loxoprofen, meloxicam, ampiroxicam, celecoxib, rofecoxib), and the like.

### (12) Others

Saccharification inhibitors (e.g., ALT-711), antidepressants (e.g., desipramine, amitriptyline, imipramine, fluoxetine, paroxetine, doxepine), antiepileptics (e.g., lamotrigine, carbamazepine), antiarrhythmic drug (e.g., mexiletine), acetylcholine receptor ligands (e.g., ABT-594), endothelin receptor antagonists (e.g., ABT-627), monoamine uptake inhibitors (e.g., tramadol), indolamine uptake inhibitors (e.g., fluoxetine, paroxetine), narcotic analgesics (e.g., morphine), GABA receptor agonists (e.g., gabapentin), GABA uptake inhibitor (e.g., tiagabine), α2 receptor agonists (e.g., clonidine), local analgesics (e.g., capsaicin), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatriptan), serotonin receptor antagonists (e.g., cyproheptadine hydrochloride, ondansetron), serotonin uptake inhibitors (e.g., fluvoxamine maleate, fluoxetine, paroxetine), sleep-inducing drugs (e.g., triazolam, zolpidem), anticholinergic drugs, α1 receptor blockers (e.g., tamsulosin), muscle relaxants (e.g., baclofen), agents for preventing or treating Alzheimer's disease (e.g., donepezil, rivastigmine, galanthamine), agents for treating Parkinson's disease (e.g., L-dopa), agents for preventing or treating multiple sclerosis (e.g., interferon (β-1a), histamine H1 receptor inhibitors (e.g., promethazine chloride), proton pump inhibitors (e.g., lansoprazole, omeprazole, rabeprazole or salts thereof (e.g., sodium salt)), NK-2 receptor antagonists, agents for treating HIV infectious disease (e.g., saquinavir, zidovudine, lamivudine, neviravine), agents for treating chronic obstructive lung disease (e.g., salmeterol, tiotropium bromide, cilomilast), and the like.

As the anticholinergic agent, for example, atropine, scopolamine, homatropine, tropicamide, cyclopentolate, scopolamine butylbromide, propantheline bromide, methylbenactyzium bromide, mepenzolate bromide, flavoxate, pirensevine, ipratopium bromide, trihexyphenidyl, oxybutynin, propiverine, darifenacin, tolterodine, temiverine, trospium chloride or salts thereof (e.g., atropine sulfate, scopolamine hydrobromide, homatropine hydrobromide, cyclopentolate hydrochloride, flavoxate hydrochloride, pirensevine hydrochloride, trihexyphenidyl hydrochloride, oxybutynin hydrochloride, tolterodine tartrate) and the like are used, and among these, oxybutynin, propiverine, darifenacin, tolterodine, temiverine, trospium chloride or salts thereof (e.g., oxybutynin chloride, tolterodine tartrate) are preferable. Furthermore, acetylcholinesterase inhibitors (e.g., distigmine) and the like can also be used.

As the NK-2 receptor antagonist, for example, piperidine derivatives such as GR159897, GR149861, SR48968 (saredutant), SR144190, YM35375, YM38336, ZD7944, L-743986, MDL105212A, ZD6021, MDL105172A, SCH205528, SCH62373, R-113281 and the like; perhydroisoindole derivatives such as RPR-106145 and the like; quinoline derivatives such as SB-414240 and the like; pyrolopyrimidine derivatives such as ZM-253270 and the like; pseudopeptide derivatives such as MEN11420 (nepadutant), SCH217048, L-659877, PD-147714 (CAM-2291), MEN10376, S16474 and the like; other agents such as GR100679, DNK333, GR94800, UK-224671, MEN10376, MEN10627 or salts thereof and the like, and the like are exemplified.

When the poorly water-soluble substance having a low melting point is a lipase inhibitor (preferably compound A), the concomitant drug is preferably an insulin preparation, an agent for improving insulin resistance (preferably pioglitazone or a salt thereof (preferably hydrochloride)), an α-glucosidase inhibitor (preferably voglibose), a biguanide (preferably metformin), an insulin secretagogue (preferably a sulfonylurea agent, mitiglinide or a calcium salt hydrate thereof), an HMG-CoA reductase inhibitor (preferably simvastatin, atorvastatin), and the like.

The solid preparation of the present invention containing or using in combination a poorly water-soluble substance having a low melting point and a concomitant drug includes (1) a single preparation of a pharmaceutical composition containing a poorly water-soluble substance having a low melting point and a concomitant drug, and (2) a pharmaceutical composition containing a poorly water-soluble substance having a low melting point and a concomitant drug, which are prepared independently. In the following, they are comprehensively abbreviated as the combination agent of the present invention.

The combination agent of the present invention can be formulated by subjecting a poorly water-soluble substance having a low melting point and the active ingredient of a concomitant drug, separately or simultaneously, directly or in the form of a mixture with a pharmaceutically acceptable carrier and the like, to a method similar to that of the aforementioned solid preparation of the present invention.

While the daily dose of the combination agent of the present invention varies depending on the symptom, human race, age, sex and body weight of the administration subject, administration form, the kind of active ingredient and the like, it is not particularly limited as long as the range does not cause a problem of side effects. The daily dose of the combination agent of the present invention as a total dose of a poorly water-soluble substance having a low melting point and a concomitant drug, for example, in the case of oral administration, is generally about 0.005 to 100 mg, preferably about 0.05 to 50 mg, more preferably about 0.2 to 30 mg, per 1 kg body weight of mammal, which is generally administered in 1 to 3 portions a day.

For administration of the combination agent of the present invention, the solid preparation of the present invention and a concomitant drug may be administered simultaneously, or a concomitant drug may be administered first and then the solid preparation of the present invention may be administered, or the solid preparation of the present invention may be administered first and then the concomitant drug may be administered. For administration in a staggered manner, the time difference varies depending on the active ingredient to be administered, dosage form and administration method. For example, when the concomitant drug is to be administered first, a method comprising administering the concomitant drug and then administering the solid preparation of the present invention within 1 min to 3 days, preferably within 10 min to 1 day, more preferably within 15 min to 1 hr can be mentioned. When the solid preparation of the present invention is to be administered first, a method comprising administering the solid preparation of the present invention and then administering the concomitant drug within 1 min to 1 day, preferably within 10 min to 6 hr, more preferably within 15 min to 1 hr can be mentioned.

In the combination agent of the present invention, while the content of the solid preparation of the present invention relative to the whole combination agent varies depending on the form of the combination agent, it is generally 0.3 wt% to 65 wt%, preferably 0.1 wt% to 50 wt%, more preferably about 0.5 wt% to 20 wt%.

### Examples

The present invention is explained in detail in the following by referring to Examples, which are not to be construed as limitative.

In the following Examples and Comparative Examples, the Japanese Pharmacopoeia 14th Edition compatible products or Japanese Pharmaceutical Excipients 2003 compatible products were used as various additives such as magnesium stearate and the like.

In addition, the hardness (destruction hardness) of the tablet described in the following Examples and Comparative Examples was measured using PHARMA TEST APPARATEBAU GMBH, WHT 2ME.

### Example 1

A mixed powder of compound A (660.0 g, average particle size: 20 to 50 µm), mannitol (3196 g, Merck), crystalline cellulose (591.8 g, Asahi Kasei Chemical Corporation, trade name: Ceolus, grade PH101) and light anhydrous silicic acid (50.6 g, Nippon Aerosil) was placed in a fluidized bed granulator, and the mixture was granulated while spraying an aqueous solution (2457 g) of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (210.9 g, Asahi Kasei Chemical Corporation, trade name: Ac-Di-Sol) and magnesium stearate (40.7 g, Taiheiyo Kagaku Co., Ltd.) were added to the sized powder (3911 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 13500 film-coated tablets containing compound A (30 mg), mannitol (145.5 mg), crystalline cellulose (26.9 mg), light anhydrous silicic acid (2.3 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 2

A mixed powder of compound A (1140.0 g, average particle size: 20 to 50 µm), mannitol (2187 g, Merck), crystalline cellulose (511.1 g, Asahi Kasei Chemical Corporation, trade name: Ceolus, grade PH101) and light anhydrous silicic acid (43.7 g, Nippon Aerosil) was placed in a fluidized bed granulator, and the mixture was granulated while spraying an aqueous solution (2122 g) of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (368.2 g, Asahi Kasei Chemical Corporation, trade name: Ac-Di-Sol) and magnesium stearate (71.06 g, Taiheiyo Kagaku Co., Ltd.) were added to the sized powder (6828 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 22500 film-coated tablets containing compound A (60 mg), mannitol (115.5 mg), crystalline cellulose (26.9 mg), light anhydrous silicic acid (2.3 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 3

A mixed powder of compound A (600.0 g, average particle size: 20 to 50 µm), mannitol (2910 g, Merck), crystalline cellulose (538 g, Asahi Kasei Chemical Corporation, trade name: Ceolus, grade PH101) and light anhydrous silicic acid (46 g, Nippon Aerosil) was placed in a fluidized bed granulator, and the mixture was granulated while spraying an aqueous solution (2234 g) of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (199.5 g, Asahi Kasei Chemical Corporation, trade name: Ac-Di-Sol) and magnesium stearate (38.5 g, Taiheiyo Kagaku Co., Ltd.) were added to the sized powder (3700 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 15000 film-coated tablets containing compound A (30 mg), mannitol (145.5 mg), crystalline cellulose (26.9 mg), light anhydrous silicic acid (2.3 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 4

A mixed powder of compound A (1200.0 g, average particle size: 20 to 50 µm), mannitol (2310 g, Merck), crystalline cellulose (538 g, Asahi Kasei Chemical Corporation, trade name: Ceolus, grade PH101) and light anhydrous silicic acid (46 g, Nippon Aerosil) was placed in a fluidized bed granulator, and the mixture was granulated while spraying an aqueous solution (2234 g) of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (199.5 g, Asahi Kasei Chemical Corporation, trade name: Ac-Di-Sol) and magnesium stearate (38.5 g, Taiheiyo Kagaku Co., Ltd.) were added to the sized powder (3700 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 15000 film-coated tablets containing compound A (60 mg), mannitol (115.5 mg), crystalline cellulose (25.9 mg), light anhydrous silicic acid (2.3 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 5

A mixed powder of compound A (36.0 g, average particle size: 20 to 50 µm), lactose (390.5 g, Meggle) and crystalline cellulose (64.6 g, Asahi Kasei Chemical Corporation, trade name: Ceolus, grade PH101) was placed in a fluidized bed granulator, and the mixture was granulated while spraying an aqueous solution (209.9 g) of 8% (wt./v) hydroxypropylmethylcellulose (Shin-Etsu Chemical Co., Ltd., trade name: TC-5, grade RW) with flowing and dried to give granules. The obtained granules were passed through a 16 mesh sieve to give sized powder. Croscarmellose sodium (10.3 g, Asahi Kasei Chemical Corporation, trade name: Ac-Di-Sol) and magnesium stearate (2.0 g, Taiheiyo Kagaku Co., Ltd.) were added to the sized powder (190.3 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 600 film-coated tablets containing compound A (15 mg), lactose (162.8 mg), crystalline cellulose (26.9 mg), hydroxypropylmethylcellulose 2910 (13.9 mg), croscarmellose sodium (11.4 mg), magnesium stearate (2.2 mg), macrogol 6000 (1.6 mg), titanium oxide (1.0 mg) and diiron trioxide (0.2 mg) per tablet.

### Example 6

A mixed powder of compound A (33.0 g, average particle size: 20 to 50 µm), mannitol (160.1 g, Merck), crystalline cellulose (29.6 g, Asahi Kasei Chemical Corporation, trade name: Ceolus, grade PH101) and light anhydrous silicic acid (2.5 g, Nippon Aerosil) was placed in a fluidized bed granulator, and the mixture was granulated while spraying an aqueous solution (115.9 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (10.8 g, Asahi Kasei Chemical Corporation, trade name: Ac-Di-Sol) and magnesium stearate (2.1 g, Taiheiyo Kagaku Co., Ltd.) were added to the sized powder (200.8 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 600 film-coated tablets containing compound A (30 mg), mannitol (145.5 mg), crystalline cellulose (26.9 mg), light anhydrous silicic acid (2.3 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 7

A mixed powder of compound A (85.0 g, average particle size: 20 to 50 µm), lactose (410.8 g, Meggle) and crystalline cellulose (85.0 g, Asahi Kasei Chemical Corporation, trade name: Ceolus, grade PH101) was placed in a fluidized bed granulator, and the mixture was granulated while spraying an aqueous solution (549.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (25.5 g, Asahi Kasei Chemical Corporation, trade name: Ac-Di-Sol) and magnesium stearate (2.55 g, Taiheiyo Kagaku Co., Ltd.) were added to the sized powder (450.0 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 1200 film-coated tablets containing compound A (30 mg), lactose (147.8 mg), crystalline cellulose (26.9 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 8

A mixed powder of compound A (85.0 g, average particle size: 20 to 50 µm), mannitol (410.8 g, Roquette) and crystalline cellulose (85.0 g, Asahi Kasei Chemical Corporation, trade name: Ceolus, grade PH101) was placed in a fluidized bed granulator, and the mixture was granulated while spraying an aqueous solution (549.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (25.5 g, Asahi Kasei Chemical Corporation, trade name: Ac-Di-Sol) and magnesium stearate (2.55 g, Taiheiyo Kagaku Co., Ltd.) were added to the sized powder (450.0 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries, Ltd.), diiron trioxide and hydroxypropylmethylcellulose 2910 (Shin-Etsu Chemical Co., Ltd., trade name: TC-5, grade RW) in purified water was sprayed to give about 1200 film-coated tablets containing compound A (30 mg), mannitol (147.8 mg), crystalline cellulose (26.9 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 9

A mixed powder of compound A (85.0 g, average particle size: 20 to 50 µm), mannitol (410.8 g, Roquette) and crystalline cellulose (85.0 g, Asahi Kasei Chemical Corporation, trade name: Ceolus, grade PH101) was placed in a fluidized bed granulator, and the mixture was granulated while spraying an aqueous solution (549.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Low-substituted hydroxypropylcellulose (1.2 g, Shin-Etsu Chemical Co., Ltd., trade name: L-HPC) and magnesium stearate (0.12 g, Taiheiyo Kagaku Co., Ltd.) were added to the sized powder (21.18 g) and mixed to give granules for tabletting. The obtained granules were tableted in a tabletting machine using a punch having a diameter of 8.5 mm and a corner angle flat plane to give about 1500 plain tablets weighing 225 mg per tablet. The obtained plain tablets contained compound A (30 mg), mannitol (147.8 mg), crystalline cellulose (26.9 mg), hydroxypropylcellulose (6.7 mg), low-substituted hydroxypropylcellulose (11.4 mg) and magnesium stearate (2.2 mg) per tablet.

### Example 10

A mixed powder of compound A (85.0 g, average particle size: 20 to 50 µm), mannitol (410.8 g, Roquette) and crystalline cellulose (85.0 g, Asahi Kasei Chemical Corporation, trade name: Ceolus, grade PH101) was placed in a fluidized bed granulator, and the mixture was granulated while spraying an aqueous solution (549.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Carboxymethyl starch sodium (1.2 g) and magnesium stearate (0.12 g, Taiheiyo Kagaku Co., Ltd.) were added to the sized powder (21.18 g) and mixed to give granules for tabletting. The obtained granules were tableted in a tabletting machine using a punch having a diameter of 8.5 mm and a corner angle flat plane to give about 1200 plain tablets weighing 225 mg per tablet. The obtained plain tablets contained compound A (30 mg), mannitol (147.8 mg), crystalline cellulose (26.9 mg), hydroxypropylcellulose (6.7 mg), carboxymethyl starch sodium (11.4 mg) and magnesium stearate (2.2 mg) per tablet.

### Example 11

A mixed powder of compound A (108.0 g, average particle size: 20 to 50 µm), mannitol (207.9 g, Roquette), crystalline cellulose (48.4 g, Asahi Kasei Chemical Corporation, trade name: Ceolus, grade PH101) and light anhydrous silicic acid (Nippon Aerosil) was placed in a fluidized bed granulator, and the mixture was granulated while spraying an aqueous solution (189.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (17.1 g, Asahi Kasei Chemical Corporation, trade name: Ac-Di-Sol) and magnesium stearate (3.3 g, Taiheiyo Kagaku Co., Ltd.) were added to the sized powder (317.1 g) and mixed to give granules for tabletting. The obtained granules were tableted in a tabletting machine using a round punch having a diameter of 8 mm to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 800 film-coated tablets containing compound A (60 mg), mannitol (115.5 mg), crystalline cellulose (26.9 mg), light anhydrous silicic acid (2.3 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 12

A mixed powder of compound A (108.0 g, average particle size: 20 to 50 µm), mannitol (207.9 g, Roquette), crystalline cellulose (48.4 g, Asahi Kasei Chemical Corporation, trade name: Ceolus, grade PH101) and light anhydrous silicic acid (Nippon Aerosil) was placed in a fluidized bed granulator, and the mixture was granulated while spraying an aqueous solution (189.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (17.1 g, Asahi Kasei Chemical Corporation, trade name: Ac-Di-Sol) and magnesium stearate (3.3 g, Taiheiyo Kagaku Co., Ltd.) were added to the sized powder (317.1 g) and mixed to give granules for tabletting. The obtained granules were tableted in a tabletting machine using a punch having a diameter of 8.5 mm and a corner angle flat plane to give about 1000 plain tablets weighing 225 mg per tablet. The obtained plain tablets contained compound A (60 mg), mannitol (115.5 mg), crystalline cellulose (26.9 mg), light anhydrous silicic acid (2.3 mg), hydroxypropylcellulose (6.7 mg), croscarmellose sodium (11.4 mg) and magnesium stearate (2.2 mg) per tablet.

### Example 13

A mixed powder of compound A (120.0 g, average particle size: 20 to 50 µm), lactose (234.8 g, Meggle) and crystalline cellulose (53.8 g, Asahi Kasei Chemical Corporation, trade name: Ceolus, grade PH101) was placed in a fluidized bed granulator, and the mixture was granulated while spraying an aqueous solution (210.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (20.3 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give granules for tabletting. The obtained granules were tableted in a tabletting machine using a round punch having a diameter of 8 mm to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 600 film-coated tablets containing compound A (60 mg), lactose (117.8 mg), crystalline cellulose (26.9 mg), hydroxypropylcellulose (6.7 mg), croscarmellose sodium (11.4 mg), magnesium stearate (2.2 mg), hydroxypropylmethylcellulose 2910 (7.2 mg), macrogol 6000 (1.6 mg), titanium oxide (1.0 mg) and diiron trioxide (0.2 mg) per tablet.

### Example 14

A mixed powder of compound A (120.0 g, average particle size: 20 to 50 µm), lactose (230.2 g, Meggle), crystalline cellulose (53.8 g, Asahi Kasei Chemical Corporation, trade name: Ceolus, grade PH101) and light anhydrous silicic acid (4.6 g, Nippon Aerosil) was placed in a fluidized bed granulator, and the mixture was granulated while spraying an aqueous solution (210.0 g) of 6% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (20.0 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give granules for tabletting. The obtained granules were tableted in a tabletting machine using a round punch having a diameter of 8 mm to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 600 film-coated tablets containing compound A (60 mg), lactose (115.5 mg), crystalline cellulose (26.9 mg), light anhydrous silicic acid (2.3 mg), hydroxypropylcellulose (6.7 mg), croscarmellose sodium (11.4 mg) and magnesium stearate (2.2 mg) per tablet.

### Example 15

A mixed powder of compound A (120.0 g, average particle size: 20 to 50 µm), lactose (234.8 g, Meggle) and crystalline cellulose (53.8 g, Asahi Kasei Chemical Corporation, trade name: Ceolus, grade PH101) was placed in a fluidized bed granulator, and the mixture was granulated while spraying an aqueous solution (154.2 g) of 8% (wt./v) hydroxypropylmethylcellulose 2910 (Shin-Etsu Chemical Co., Ltd., trade name: TC-5, grade RW) with flowing and dried to give granules. The obtained granules were passed through a 16 mesh sieve to give sized powder. Croscarmellose sodium (20.0 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give granules for tabletting. The obtained granules were tableted in a tabletting machine using a punch having a diameter of 8 mm and a circle-shaped R surface to give plain tablets weighing 225 mg per tablet. The obtained plain tablets were placed in a film coating machine, and a liquid obtained by dissolving or dispersing titanium oxide, macrogol 6000 (Sanyo Chemical Industries. Ltd.), diiron trioxide and hydroxypropylmethylcellulose 2910 (Shin-Etsu Chemical Co., Ltd., trade name: TC-5, grade RW) in purified water was sprayed to give about 600 film-coated tablets containing compound A (60 mg), lactose (117.8 mg), crystalline cellulose (26.9 mg), hydroxypropylmethylcellulose 2910 (13.9 mg), croscarmellose sodium (11.4 mg), magnesium stearate (2.2 mg), macrogol 6000 (1.6 mg), titanium oxide (1.0 mg) and diiron trioxide (0.2 mg) per tablet.

### Example 16

A mixed powder of compound A (120.0 g, average particle size: 20 to 50 µm), lactose (234.8 g, Meggle) and crystalline cellulose (53.8 g, Asahi Kasei Chemical Corporation, trade name: Ceolus, grade PH101) was placed in a fluidized bed granulator, and the mixture was granulated while spraying an aqueous solution (154.2 g) of 8% (wt./v) hydroxypropylmethylcellulose 2910 (Shin-Etsu Chemical Co., Ltd., trade name: TC-5, grade RW) with flowing and dried to give granules. The obtained granules were passed through a 16 mesh sieve to give sized powder. Croscarmellose sodium (20.0 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give a mixed powder. Ascorbic acid (0.25 g) was added to the obtained mixed powder (11.25 g) and mixed to give granules for tabletting. The obtained granules were tableted in a tabletting machine using a punch having a diameter of 8 mm and a circle-shaped R surface to give about 1200 plain tablets weighing 230 mg per tablet. The obtained plain tablets contained compound A (60 mg), lactose (117.8 mg), crystalline cellulose (26.9 mg), hydroxypropylmethylcellulose 2910 (6.7 mg), ascorbic acid (5 mg), croscarmellose sodium (11.4 mg) and magnesium stearate (2.2 mg) per tablet.

### Example 17

A mixed powder of compound A (120.0 g, average particle size: 20 to 50 µm), lactose (234.8 g, Meggle) and crystalline cellulose (53.8 g, Asahi Kasei Chemical Corporation, trade name: Ceolus, grade PH101) was placed in a fluidized bed granulator, and the mixture was granulated while spraying an aqueous solution (154.2 g) of 8% (wt./v) hydroxypropylmethylcellulose 2910 (Shin-Etsu Chemical Co., Ltd., trade name: TC-5, grade RW) with flowing and dried to give granules. The obtained granules were passed through a 16 mesh sieve to give sized powder. Croscarmellose sodium (20.0 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give a mixed powder. Sodium ascorbate (0.25 g) was added to the obtained mixed powder (11.25 g) and mixed to give granules for tabletting. The obtained granules were tableted in a tabletting machine using a punch having a diameter of 8 mm and a circle-shaped R surface to give about 1200 plain tablets weighing 230 mg per tablet. The obtained plain tablets contained compound A (60 mg), lactose (117.8 mg), crystalline cellulose (26.9 mg), hydroxypropylmethylcellulose 2910 (6.7 mg), sodium ascorbate (5 mg), croscarmellose sodium (11.4 mg) and magnesium stearate (2.2 mg) per tablet.

### Example 18

A mixed powder of compound A (120.0 g, average particle size: 20 to 50 µm), lactose (234.8 g, Meggle) and crystalline cellulose (53.8 g, Asahi Kasei Chemical Corporation, trade name: Ceolus, grade PH101) was placed in a fluidized bed granulator, and the mixture was granulated while spraying an aqueous solution (154.2 g) of 8% (wt./v) hydroxypropylmethylcellulose 2910 (Shin-Etsu Chemical Co., Ltd., trade name: TC-5, grade RW) with flowing and dried to give granules. The obtained granules were passed through a 16 mesh sieve to give sized powder. Croscarmellose sodium (20.0 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give a mixed powder. Erysorbic acid (0.25 g) was added to the obtained mixed powder (11.25 g) and mixed to give granules for tabletting. The obtained granules were tableted in a tabletting machine using a punch having a diameter of 8 mm and a circle-shaped R surface to give about 1200 plain tablets weighing 230 mg per tablet. The obtained plain tablets contained compound A (60 mg), lactose (117.8 mg), crystalline cellulose (26.9 mg), hydroxypropylmethylcellulose 2910 (6.7 mg), erysorbic acid (5 mg), croscarmellose sodium (11.4 mg) and magnesium stearate (2.2 mg) per tablet.

### Example 19

A mixed powder of compound A (120.0 g, average particle size: 20 to 50 µm), lactose (234.8 g, Meggle) and crystalline cellulose (53.8 g, Asahi Kasei Chemical Corporation, trade name: Ceolus, grade PH101) was placed in a fluidized bed granulator, and the mixture was granulated while spraying an aqueous solution (154.2 g) of 8% (wt./v) hydroxypropylmethylcellulose 2910 (Shin-Etsu Chemical Co., Ltd., trade name: TC-5, grade RW) with flowing and dried to give granules. The obtained granules were passed through a 16 mesh sieve to give sized powder. Croscarmellose sodium (20.0 g, Asahi Kasei Chemical Corporation, trade name: Ac-Di-Sol) and magnesium stearate (3.9 g, Taiheiyo Kagaku Co., Ltd.) were added to the sized powder (370.0 g) and mixed to give a mixed powder. 2,6-di-t-Butyl-4-methylphenol (0.25 g) was added to the obtained mixed powder (11.25 g) and mixed to give granules for tabletting. The obtained granules were tableted in a tabletting machine using a punch having a diameter of 8 mm and a circle-shaped R surface to give about 1200 plain tablets weighing 230 mg per tablet. The obtained plain tablets contained compound A (60 mg), lactose (117.8 mg), crystalline cellulose (26.9 mg), hydroxypropylmethylcellulose 2910 (6.7 mg), 2,6-di-t-butyl-4-methylphenol (5 mg), croscarmellose sodium (11.4 mg) and magnesium stearate (2.2 mg) per tablet.

### Example 20

Compound A (45.0 g, average particle size: 20 to 50 µm), mannitol (220.5 g, Merck) and crystalline cellulose (40.2 g, Asahi Kasei Chemical Corporation, trade name: Ceolus, grade PH101) were mixed and placed in a fluidized bed granulator, and the mixture was granulated while spraying an aqueous solution (214.2 g) of 5% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (14.6 g, Asahi Kasei Chemical Corporation, trade name: Ac-Di-Sol) and magnesium stearate (2.9 g, Taiheiyo Kagaku Co., Ltd.) were added to the sized powder (273.8 g) and mixed to give a mixed powder. The obtained mixed powder was tableted in a tabletting machine using a punch having a diameter of 6.5 mm and a circle-shaped R surface to give about 2000 plain tablets weighing 112.0 mg per tablet. The obtained plain tablets contained compound A (15 mg), mannitol (73.5 mg), crystalline cellulose (13.4 mg), hydroxypropylcellulose (3.4 mg), croscarmellose sodium (5.6 mg) and magnesium stearate (1.1 mg) per tablet.

### Example 21

Compound A (75.0 g, average particle size: 20 to 50 µm), mannitol (146.3 g, Merck) and crystalline cellulose (33.5 g, Asahi Kasei Chemical Corporation, trade name: Ceolus, grade PH101) were mixed and placed in a fluidized bed granulator, and the mixture was granulated while spraying an aqueous solution (161.5 g) of 5% (wt./v) hydroxypropylcellulose (Nippon Soda Co., Ltd., HPC, grade L) with flowing and dried to give granules. The obtained granules were passed through a 16 mesh sieve to give sized powder. Croscarmellose sodium (12 g, Asahi Kasei Chemical Corporation, trade name: Ac-Di-Sol) and magnesium stearate (2.4 g, Taiheiyo Kagaku Co., Ltd.) were added to the sized powder (226.4 g) and mixed to give a mixed powder. The obtained mixed powder was tableted in a tabletting machine using a punch having a diameter of 8 mm and a corner angle flat plane to give about 1250 plain tablets weighing 224.0 mg per tablet. The obtained plain tablets contained compound A (30 mg), mannitol (58.5 mg), crystalline cellulose (13.4 mg), hydroxypropylcellulose (3.4 mg), croscarmellose sodium (5.6 mg) and magnesium stearate (1.1 mg) per tablet.

### Example 22

Compound A (660.0 g, average particle size: 20 to 50 µm), mannitol (3226 g, Merck) and crystalline cellulose (589.6 g, Asahi Kasei Chemical Corporation, trade name: Ceolus, grade PH101) were mixed and placed in a fluidized bed granulator, and the mixture was granulated while spraying a liquid (2490 g) obtained by suspending yellow ferric oxide (4.4 g) in an aqueous solution of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (207.2 g, Asahi Kasei Chemical Corporation, trade name: Ac-Di-Sol) and magnesium stearate (40.7 g, Taiheiyo Kagaku Co., Ltd.) were added to the sized powder (3896 g) and mixed to give a mixed powder. The obtained mixed powder was tableted in a tabletting machine using a punch having a diameter of 6.5 mm and a corner angle flat plane to give about 20000 plain tablets weighing 112.0 mg per tablet. The obtained plain tablets contained compound A (15 mg), mannitol (73.4 mg), crystalline cellulose (13.4 mg), yellow ferric oxide (0.1 mg), hydroxypropylcellulose (3.5 mg), croscarmellose sodium (5.6 mg) and magnesium stearate (1.1 mg) per tablet.

### Example 23

Compound A (1320 g, average particle size: 20 to 50 µm), mannitol (2563 g, Merck) and crystalline cellulose (589.6 g, Asahi Kasei Chemical Corporation, trade name: Ceolus, grade PH101) were mixed and placed in a fluidized bed granulator, and the mixture was granulated while spraying a liquid (2490 g) obtained by suspending yellow ferric oxide (4.4 g) in an aqueous solution of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (207.2 g, Asahi Kasei Chemical Corporation, trade name: Ac-Di-Sol) and magnesium stearate (40.7 g, Taiheiyo Kagaku Co., Ltd.) were added to the sized powder (3896 g) and mixed to give a mixed powder. The obtained mixed powder was tableted in a tabletting machine using a punch having a diameter of 6.5 mm and a corner angle flat plane to give about 20000 plain tablets weighing 112.0 mg per tablet. The obtained plain tablets contained compound A (30 mg), mannitol (58.4 mg), crystalline cellulose (13.4 mg), yellow ferric oxide (0.1 mg), hydroxypropylcellulose (3.5 mg), croscarmellose sodium (5.6 mg) and magnesium stearate (1.1 mg) per tablet.

In addition, the obtained plain tablet had a hardness of 70N.

### Example 24

Compound A (1320 g, average particle size: 20 to 50 (m), mannitol (2563 g, Merck) and crystalline cellulose (589.6 g, Asahi Kasei Chemical Corporation, trade name: Ceolus, grade PH101) were mixed and placed in a fluidized bed granulator, and the mixture was granulated while spraying a liquid (2495 g) obtained by suspending yellow ferric oxide (4.4 g) in an aqueous solution of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder. Croscarmellose sodium (207.2 g, Asahi Kasei Chemical Corporation, trade name: Ac-Di-Sol) and magnesium stearate (40.7 g, Taiheiyo Kagaku Co., Ltd.) were added to the sized powder (3896 g) and mixed to give a mixed powder. The obtained mixed powder was tableted in a tabletting machine using a punch having a diameter of 8.5 mm and a corner angle flat plane to give about 10000 plain tablets weighing 224.0 mg per tablet. The obtained plain tablets contained compound A (60 mg), mannitol (116.8 mg), crystalline cellulose (26.8 mg), yellow ferric oxide (0.2 mg), hydroxypropylcellulose (7.0 mg), croscarmellose sodium (11.2 mg) and magnesium stearate (2.2 mg) per tablet.

### Example 25

Compound A (1320 g, average particle size: 20 to 50 (m), mannitol (2563 g, Merck) and crystalline cellulose (589.6 g, Asahi Kasei Chemical Corporation, trade name: Ceolus, grade PH101) were mixed and placed in a fluidized bed granulator, and the mixture was granulated while spraying a liquid (2495 g) obtained by suspending yellow ferric oxide (4.4 g) in an aqueous solution of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder A. On the other hand, mannitol (3306 g, Merck) and crystalline cellulose (501.2 g, Asahi Kasei Chemical Corporation, trade name: Ceolus, grade PH101) were mixed, placed in a fluidized bed granulator, and granulated while spraying a liquid (2117 g) obtained by suspending yellow ferric oxide (4.4 g) in an aqueous solution of 6% (wt./v) hydroxypropylcellulose (Nippon Soda Co., Ltd., HPC, grade L) with flowing and dried to give granules. The obtained granules were milled in a power mill equipped with a 1.5 mmφ punching screen to give sized powder P.

The sized powder A (731.8 g) and the sized powder P (2196 g) were uniformly mixed, croscarmellose sodium (155.7 g, Asahi Kasei Chemical Corporation, trade name: Ac-Di-Sol) and magnesium stearate (30.6 g, Taiheiyo Kagaku Co., Ltd.) were added to the sized powder and mixed to give a mixed powder. The obtained mixed powder was tableted in a tabletting machine using a punch having a diameter of 6.5 mm and a corner angle flat plane to give about 10000 plain tablets weighing 112.0 mg per tablet. The obtained plain tablets contained compound A (7.5 mg), mannitol (80.9 mg), crystalline cellulose (13.4 mg), yellow ferric oxide (0.1 mg), hydroxypropylcellulose (3.4 mg), croscarmellose sodium (5.6 mg) and magnesium stearate (1.1 mg) per tablet.

### Example 26

The sized powder A (1464 g) of Example 25 and the sized powder P (1464 g) were uniformly mixed, croscarmellose sodium (155.7 g, Asahi Kasei Chemical Corporation, trade name: Ac-Di-Sol) and magnesium stearate (30.6 g, Taiheiyo Kagaku Co., Ltd.) were added to the sized powder and mixed to give a mixed powder. The obtained mixed powder was tableted in a tabletting machine using a punch having a diameter of 6.5 mm and a corner angle flat plane to give about 10000 plain tablets weighing 112.0 mg per tablet. The obtained plain tablets contained compound A (15.0 mg), mannitol (73.4 mg), crystalline cellulose (13.4 mg), yellow ferric oxide (0.1 mg), hydroxypropylcellulose (3.4 mg), croscarmellose sodium (5.6 mg) and magnesium stearate (1.1 mg) per tablet.

### Example 27

Compound A (18000 g), mannitol (34980 g, Roquette) and crystalline cellulose (8040 g, Asahi Kasei Chemical Corporation, trade name: Ceolus, grade PH101) were mixed and placed in a fluidized bed granulator, and the mixture was granulated while spraying a mixture of a solution of hydroxypropylcellulose (2040 g, Nippon Soda Co., Ltd., HPC, grade L) in purified water (24 L) and a suspension of yellow ferric oxide (60 g) in purified water (7.86 L) with flowing and dried to give granules. The obtained granules were milled in a power mill equipped with a 1.5 mmφ punching screen to give sized powder A.

On the other hand, mannitol (52980 g) and crystalline cellulose (8040 g, Asahi Kasei Chemical Corporation, trade name: Ceolus, grade PH101) were mixed and placed in a fluidized bed granulator, and the mixture was granulated while spraying a mixture of a solution of hydroxypropylcellulose (4080 g, Nippon Soda Co., Ltd., HPC, grade L) in purified water (54.4 L) and a suspension of yellow ferric oxide (120 g) in purified water (9.32 L) with flowing and dried to give granules. The obtained granules were milled in a power mill equipped with a 1.5 mmφ punching screen to give sized powder P.

The sized powder A (14990 g) and the sized powder P (44970 g) were uniformly mixed, croscarmellose sodium (3249 g, trade name: Ac-Di-Sol) and magnesium stearate (627 g, Taiheiyo Kagaku Co., Ltd.) were added to the sized powder and mixed to give a mixed powder. The obtained mixed powder was tableted in a tabletting machine using a punch having a diameter of 6.5 mm and a corner angle flat plane to give about 550000 plain tablets weighing 112.0 mg per tablet. The obtained plain tablets contained compound A (7.5 mg), mannitol (80.8 mg), crystalline cellulose (13.4 mg), yellow ferric oxide (0.1 mg), hydroxypropylcellulose (3.4 mg), croscarmellose sodium (5.7 mg) and magnesium stearate (1.1 mg) per tablet.

### Example 28

The sized powder A (29980 g) obtained in Example 27 and the sized powder P (29980 g) were uniformly mixed, croscarmellose sodium (3249 g, trade name: Ac-Di-Sol) and magnesium stearate (627 g, Taiheiyo Kagaku Co., Ltd.) were added to the sized powder and mixed to give a mixed powder. The obtained mixed powder was tableted in a tabletting machine using a punch having a diameter of 6.5 mm and a corner angle flat plane to give about 550000 plain tablets weighing 112.0 mg per tablet. The obtained plain tablets contained compound A (15.0 mg), mannitol (73.3 mg), crystalline cellulose (13.4 mg), yellow ferric oxide (0.1 mg), hydroxypropylcellulose (3.4 mg), croscarmellose sodium (5.7 mg) and magnesium stearate (1.1 mg) per tablet.

### Example 29

Compound A (18000 g), mannitol (34980 g, Roquette) and crystalline cellulose (8040 g, Asahi Kasei Chemical Corporation, trade name: Ceolus, grade PH101) were mixed and placed in a fluidized bed granulator, and the mixture was granulated while spraying a mixture of a solution of hydroxypropylcellulose (2040 g, Nippon Soda Co., Ltd., HPC, grade L) in purified water (24 L) and a suspension of yellow ferric oxide (60 g) in purified water (7.86 L) with flowing and dried to give granules. The obtained granules were milled in a power mill equipped with a 1.5 mmφ punching screen to give sized powder. The obtained sized powder (55760 g), croscarmellose sodium (3021 g, trade name: Ac-Di-Sol) and magnesium stearate (583 g, Taiheiyo Kagaku Co., Ltd.) were added to the sized powder and mixed to give a mixed powder. The obtained mixed powder was tableted in a tabletting machine using a punch having a diameter of 6.5 mm and a corner angle flat plane to give about 510000 plain tablets weighing 112.0 mg per tablet. The obtained plain tablets contained compound A (30 mg), mannitol (58.3 mg), crystalline cellulose (13.4 mg), yellow ferric oxide (0.1 mg), hydroxypropylcellulose (3.4 mg), croscarmellose sodium (5.7 mg) and magnesium stearate (1.1 mg) per tablet.

### Example 30

Compound A (18000 g), mannitol (34980 g, Roquette) and crystalline cellulose (8040 g, Asahi Kasei Chemical Corporation, trade name: Ceolus, grade PH101) were mixed and placed in a fluidized bed granulator, and the mixture was granulated while spraying a mixture of a solution of hydroxypropylcellulose (4080 g, Nippon Soda Co., Ltd., HPC, grade L) in purified water (54 L) and a suspension of yellow ferric oxide (120 g) in purified water (9.72 L) with flowing and dried to give granules. The obtained granules were milled in a power mill equipped with a 1.5 mmφ punching screen to give sized powder. This operation was repeated twice. The obtained sized powder (111500 g), croscarmellose sodium (6042 g, trade name: Ac-Di-Sol) and magnesium stearate (1166 g, Taiheiyo Kagaku Co., Ltd.) were added to the sized powder (3896 g) and mixed to give a mixed powder. The obtained mixed powder was tableted in a tabletting machine using a punch having a diameter of 8.5 mm and a corner angle flat plane to give about 510000 plain tablets weighing 224.0 mg per tablet. The obtained plain tablets contained compound A (60 mg), mannitol (116.6 mg), crystalline cellulose (26.8 mg), yellow ferric oxide (0.2 mg), hydroxypropylcellulose (6.8 mg), croscarmellose sodium (11.4 mg) and magnesium stearate (2.2 mg) per tablet.

### Example 31

The sized powder (2.104 g) obtained in Example 29 and magnesium stearate (0.022 g, Taiheiyo Kagaku Co., Ltd.) were mixed to give a mixed powder. The obtained mixed powder was tableted in a tabletting machine using a punch having a diameter of 6.5 mm and a corner angle flat plane to give about 20 plain tablets weighing 106.3 mg per tablet. The obtained plain tablets contained compound A (30 mg), mannitol (58.3 mg), crystalline cellulose (13.4 mg), yellow ferric oxide (0.1 mg), hydroxypropylcellulose (3.4 mg) and magnesium stearate (1.1 mg) per tablet.

In addition, the obtained plain tablet had a hardness of 52N, and the sufficient hardness of the plain tablet was confirmed.

### Comparative Example 1

Compound A (94.9 g), mannitol (184.5 g) and crystalline cellulose (9.5 g, Asahi Kasei Chemical Corporation, trade name: Ceolus, grade PH101) were mixed and placed in a fluidized bed granulator, and the mixture was granulated while spraying a suspension of yellow ferric oxide (0.3 g) in a liquid obtained by dissolving hydroxypropylcellulose (10.8 g, Nippon Soda Co., Ltd., HPC, grade L) in purified water (204.4 mL) with flowing and dried to give granules. The obtained granules were passed through a 16 mesh sieve to give sized powder. Magnesium stearate (0.018 g, Taiheiyo Kagaku Co., Ltd.) was added to the sized powder (1.896 g) and mixed to give a mixed powder. The obtained mixed powder was tableted in a tabletting machine using a punch having a diameter of 6.5 mm and a corner angle flat plane to give about 20 plain tablets weighing 95.7 mg per tablet. The obtained plain tablets contained compound A (30 mg), mannitol (58.3 mg), crystalline cellulose (3 mg), hydroxypropylcellulose (3.4 mg), yellow ferric oxide (0.1 mg) and magnesium stearate (0.9 mg) per tablet.

In addition, the obtained plain tablet had a hardness of 57N.

### Comparative Example 2

Compound A (85.5 g), mannitol (102.3 g) and crystalline cellulose (102.3 g, Asahi Kasei Chemical Corporation, trade name: Ceolus, grade PH101) were mixed and placed in a fluidized bed granulator, and the mixture was granulated while spraying a suspension of yellow ferric oxide (0.3 g) in a liquid obtained by dissolving hydroxypropylcellulose (9.7 g, Nippon Soda Co., Ltd., HPC, grade L) in purified water (184 mL) with flowing and dried to give granules. The obtained granules were passed through a 16 mesh sieve to give sized powder. Magnesium stearate (0.03 g, Taiheiyo Kagaku Co., Ltd.) was added to the sized powder (2.106 g) and mixed to give a mixed powder. The obtained mixed powder was tableted in a tabletting machine using a punch having a diameter of 6.5 mm and a corner angle flat plane to give about 20 plain tablets weighing 106.8 mg per tablet. The obtained plain tablets contained compound A (30 mg), mannitol (35.9 mg), crystalline cellulose (35.9 mg), hydroxypropylcellulose (3.4 mg), yellow ferric oxide (0.1 mg) and magnesium stearate (1.1 mg) per tablet.

In addition, the obtained plain tablet had a hardness of 60N.

### Experimental Example 1

The tablets obtained in Examples and Comparative Examples were subjected to the disintegration test described in the 14^{th} revised Japanese Pharmacopoeia, and the disintegration time was measured. As a test solution, water was used and an auxiliary plate was not used. The results are shown in Table 1. The disintegration time in the Table is an average value of 6 tablets.

**Table 1**

| sample | Comparative Example 1 | Example 31 |
|---|---|---|
| disintegration time (min) | not less than 30 | 3.0 |

As shown in Table 1, the disintegration time of the tablet of Comparative Example 1 having a weight ratio of saccharide/cellulose of 19.4 and a celluloses content of 3.1 wt% was not less than 30 min, but that of the tablet of Example 31 having a weight ratio of saccharide/cellulose of 4.35 and a celluloses content of 12.6 wt% was 3.0 min. That is, the solid preparation of the present invention was disintegrated in a short time, showing the superior disintegration property possessed thereby.

### Experimental Example 2

The tablets obtained in Example 23 were preserved for a given period and the disintegration time was measured in the same manner as in Experimental Example 1.

As a result, the disintegration time (average of 5 tablets each) of "initial (before preservation)", "after preservation in tight sealed glass bottle with desiccant at 40°C for 6 months" and "after open-seal preservation in the environment of relative humidity (RH) 75% at 40°C for 6 months" was 5.6 min, 4.8 min and 4.6 min, respectively.

That is, the solid preparation of the present invention showed equivalent disintegration time before preservation and after long-term preservation, thus establishing the superior preservation stability.

### Experimental Example 3

The tablets obtained in Examples and Comparative Examples were preserved under an open-seal condition in the environment of 40°C/75% RH for 3 days and changes in the appearance after preservation were evaluated. The changes in the appearance were evaluated by measuring the thickness of the tablets with a thickness gauge and comparing the thickness with that of the tablets before preservation. The results are shown in Table 2. The thickness in the Table is an average of 5 tablets.

**Table 2**

| sample | Comparative Example 2 | Example 31 |
|---|---|---|
| thickness of tablet (initial, mm) | 2.78 | 2.74 |
| thickness of (after preservation at 40°C/75% RH, mm) | 2.86 | 2.76 |

As shown in Table 2, the thickness of the tablet of Comparative Example 2 having a weight ratio of saccharide/cellulose of 1 and a celluloses content of 33.6 wt% increased by 0.08 mm, but that of the tablet of Example 31 having a weight ratio of saccharide/cellulose of 4.35 and a celluloses content of 12.6 wt% showed an increase of 0.02 mm. In addition, the tablet of Comparative Example 2 and the tablet of Example 31 had similar hardness and thickness.

That is, the solid preparation of the present invention showed superior preservation stability (suppression of swelling of preparation due to moisture absorption) as compared to the control preparation having equivalent hardness and thickness.

### Industrial Applicability

In the solid preparation of the present invention, coagulation, melting, melt adhesion and the like of a poorly water-soluble substance having a low melting point, which are generally observed during production and preservation, are suppressed. Therefore, the solid preparation of the present invention is superior in the disintegration property and release property of the poorly water-soluble substance having a low melting point, after oral administration.

Moreover, the solid preparation of the present invention is superior in the stability during production and preservation even when a poorly water-soluble substance having a low melting point is contained in a large amount, and also superior in the disintegration property and release property of the poorly water-soluble substance having a low melting point, after oral administration.

Since the production method of the present invention can be performed under temperature conditions at not more than the melting point of the poorly water-soluble substance having a low melting point, the poorly water-soluble substance having a low melting point does not require heat-melting. Therefore, the production method of the present invention does not disintegrate a poorly water-soluble substance having a low melting point and is extremely useful as a convenient production method of a solid preparation.

## Claims

1. A solid preparation having the following characteristics 1) to 3):
1) containing 2-hexadecyloxy-6-methyl-4H-3,1-benzoxazin-4-one or a salt thereof, a saccharide, and a cellulose selected from a crystalline cellulose and a low-substituted hydroxypropylcellulose,
2) a saccharide/cellulose weight ratio exceeding 2,
3) a cellulose content of not less than 5 wt%.

2. The solid preparation of claim 1, wherein the 2-hexadecyloxy-6-methyl-4H-3,1-benzoxazin-4-one or salt thereof has an average particle size of 1 to 100 µm.

3. The solid preparation of claim 1, wherein the saccharide is a sugar alcohol.

4. The solid preparation of claim 3, wherein the sugar alcohol is mannitol.

5. The solid preparation of claim 1, wherein the cellulose is a crystalline cellulose.

6. The solid preparation of claim 1, wherein the content of the 2-hexadecyloxy-6-methyl-4H-3,1-benzoxazin-4-one or salt thereof is 5 to 60 wt%.

7. The solid preparation of claim 1, wherein the content of the saccharide is 30 to 75 wt%.

8. The solid preparation of claim 1, wherein the content of the cellulose is 5 to 15 wt%.

9. The solid preparation of claim 1, wherein the weight ratio of saccharide/cellulose is 3 to 9.

10. The solid preparation of claim 1, which is a tablet.

11. The solid preparation of claim 1, which has a disintegration time in water at 37°C of within 30 min.

12. A production method of the solid preparation of claim 1, which comprises granulating a mixture of 2-hexadecyloxy-6-methyl-4H-3,1-benzoxazin-4-one or a salt thereof, a saccharide and a cellulose selected from a crystalline cellulose and a low-substituted hydroxypropylcellulose.

13. The method of claim 12, wherein the granulation is performed using a fluidized bed granulator.

## Patentansprüche

1. Feste Zubereitung mit den folgenden Kennzeichen 1) bis 3):
1) 2-Hexadecyloxy-6-methyl-4H-3,1-benzoxazin-4-on oder ein Salz davon, ein Saccharid und eine Cellulose, ausgewählt aus einer kristallinen Cellulose und einer niedrigsubstituierten Hydroxypropylcellulose, enthaltend,
2) einem Saccharid/Cellulose Gewichtsverhältnis, welches 2 überschreitet,
3) einem Cellulosegehalt von nicht weniger als 5 Gew.-%.

2. Feste Zubereitung nach Anspruch 1, worin das 2-Hexadecyloxy-6-methyl-4H-3,1-benzoxazin-4-on oder Salz davon eine durchschnittliche Partikelgröße von 1 bis 100 µm hat.

3. Feste Zubereitung nach Anspruch 1, worin das Saccharid ein Zuckeralkohol ist.

4. Feste Zubereitung nach Anspruch 3, worin der Zuckeralkohol Mannit ist.

5. Feste Zubereitung nach Anspruch 1, worin die Cellulose eine kristalline Cellulose ist.

6. Feste Zubereitung nach Anspruch 1, worin der Gehalt des 2-Hexadecyloxy-6-methyl-4H-3,1-benzoxazin-4-ons oder Salzes davon 5 bis 60 Gew.-% ist.

7. Feste Zubereitung nach Anspruch 1, worin der Gehalt des Saccharids 30 bis 75 Gew.-% ist.

8. Feste Zubereitung nach Anspruch 1, worin der Gehalt der Cellulose 5 bis 15 Gew.-% ist.

9. Feste Zubereitung nach Anspruch 1, worin das Gewichtsverhältnis von Saccharid/Cellulose 3 bis 9 ist.

10. Feste Zubereitung nach Anspruch 1, welche eine Tablette ist.

11. Feste Zubereitung nach Anspruch 1, welche eine Zerfallsgeschwindigkeit in Wasser bei 37°C von innerhalb 30 Min. hat.

12. Herstellungsverfahren der festen Zubereitung nach Anspruch 1, welches Granulieren eines Gemisches aus 2-Hexadecyloxy-6-methyl-4H-3,1-benzoxazin-4-on oder eines Salzes davon, eines Saccharids und einer Cellulose, ausgewählt aus einer kristallinen Cellulose und einer niedrigsubstituierten Hydroxypropylcellulose, umfasst.

13. Verfahren nach Anspruch 12, wobei die Granulierung unter Verwendung eines Wirbelschichtgranulators durchgeführt wird.

## Revendications

1. Préparation solide ayant les caractéristiques 1) à 3) suivantes :
1) elle contient de la 2-hexadécyloxy-6-méthyl-4H-3,1-benzoxazin-4-one ou un sel de celle-ci, un saccharide et une cellulose choisie parmi une cellulose cristalline et une hydroxypropylcellulose à faible degré de substitution,
2) un rapport en poids de saccharide/cellulose dépassant 2,
3) une teneur en cellulose non inférieure à 5% en poids.

2. Préparation solide selon la revendication 1, dans laquelle la 2-hexadécyloxy-6-méthyl-4H-3,1-benzoxazin-4-one ou un sel de celle-ci a une taille moyenne de particule de 1 à 100 µm.

3. Préparation solide selon la revendication 1, dans laquelle le saccharide est un alcool de sucre.

4. Préparation solide selon la revendication 3, dans laquelle l'alcool de sucre est le mannitol.

5. Préparation solide selon la revendication 1, dans laquelle la cellulose est une cellulose cristalline.

6. Préparation solide selon la revendication 1, dans laquelle la teneur en 2-hexadécyloxy-6-méthyl-4H-3,1-benzoxazin-4-one ou un sel de celle-ci est de 5 à 60% en poids.

7. Préparation solide selon la revendication 1, dans laquelle la teneur en saccharide est de 30 à 75% en poids.

8. Préparation solide selon la revendication 1, dans laquelle la teneur en cellulose est de 5 à 15% en poids.

9. Préparation solide selon la revendication 1, dans laquelle le rapport en poids du saccharide/cellulose est de 3 à 9.

10. Préparation solide selon la revendication 1, qui est un comprimé.

11. Préparation solide selon la revendication 1, qui a une durée de désagrégation dans l'eau à 37°C de l'ordre de 30 min.

12. Procédé de production de la préparation solide selon la revendication 1, qui comprend la granulation d'un mélange de 2-hexadécyloxy-6-méthyl-4H-3,1-benzoxazin-4-one ou un sel de celle-ci, un saccharide et une cellulose choisie parmi une cellulose cristalline et une hydroxypropylcellulose à faible degré de substitution.

13. Procédé selon la revendication 12, dans lequel la granulation est réalisée en utilisant un granulateur à lit fluidisé.
